# EUROPÄISCHE PATENTSCHRIFT

(11) **EP 2 568 939 B1**
(45) Veröffentlichungstag und Bekanntmachung des Hinweises auf die Patenterteilung: **18.03.2015**
(21) Anmeldenummer: 11719527.1
(22) Anmeldetag: 10.05.2011
(51) Int. Cl.: A61F 13/00, A61L 15/00

(54) **WUNDAUFLAGE**
WOUND DRESSING
PANSEMENT POUR PLAIE

(30) Priorität: 11.05.2010 DE 102010020050
(43) Veröffentlichungstag der Anmeldung: 20.03.2013
(73) Patentinhaber: IVF Hartmann AG, 8212 Neuhausen am Rheinfall (CH)
(72) Erfinder: KNILL, Esther, CH-8405 Winterthur (CH); BRUGSISSER, Regina, CH-8400 Winterthur (CH)
(74) Vertreter: DREISS Patentanwälte PartG mbB
(86) Internationale Anmeldenummer: PCT/EP2011/057491
(87) Internationale Veröffentlichungsnummer: WO 2011/141454

(56) Entgegenhaltungen:
- WO-A1-2010/024928
- WO-A2-03/039602
- US-A1- 2004 142 019
- US-A1- 2007 255 193
- US-A1- 2008 082 059

## Beschreibung

Die Erfindung betrifft eine Wundauflage zur Wundbehandlung im feuchten oder feuchtnassen Milieu, mit einem faservliesbasierten Saug-/Spülkörper, in dem superabsorbierendes Material verteilt aufgenommen ist, wobei der Saug-/Spülkörper herstellerseitig mit einer salzhaltigen wässrigen Lösung, insbesondere Ringerlösung, vorzugsweise bis zur Sättigung, beaufschlagt ist, und mit einer die äußeren Sichtseiten der Wundauflage bildenden Umhüllung, wobei auf der wundabgewandten Seite des Saug-/Spülkörpers eine verdunstungshemmende Folienschicht vorgesehen sein kann.

Eine derartige Wundauflage ist aus EP 0 594 034 B1 der Anmelderin bekannt. Es handelt sich hierbei um eine wundkissenartige oder kompressenartige Wundauflage, die auf eine Wunde aufbringbar ist oder auch zum Austamponieren tiefer Wunden verwendet werden kann. Der Saug-/Spülkörper wird herstellerseitig vorzugsweise bis zur Sättigung mit einer salzhaltigen wässrigen Lösung beaufschlagt, welche das superabsorbierende Material quellen und in einen gelartigen Zustand übergehen lässt. Dies verleiht dem Saug-/Spülkörper eine duale Funktion bei Wunden mit starker Exsudation. Wundsekrete werden einschließlich ihrer kritischen Bestandteile wie Keime durch den Saug-/Spülkörper aktiv aufgenommen und darin gehalten, wobei der Saug-/Spülkörper im Gegenzug die salzhaltige wässrige Lösung an die Wunde abgibt und somit ein feuchtes Wundmilieu schafft oder unterstützt. Hierdurch wird die Wundreinigung und eine positive Wundkonditionierung unterstützt und somit die Heilung positiv beeinflusst. Man spricht hier von interaktiver Nasstherapie, die insbesondere bei schlecht heilenden Wunden, bei klinisch manifest infizierten Wunden oder bei chronischen Wunden mit unterschiedlicher Genese, wie diabetisches Gangrän, Dekubitalulcus oder Ulcus cruris bevorzugt Anwendung findet.

Bei der vorerwähnten Ringerlösung handelt es sich typischerweise um eine wässrige Lösung mit Natriumchlorid, Kaliumchlorid und Calciumchlorid (insbesondere 8,6 g NaCl, 0,3 g KCl und 0,33 g CaCl₂ je Liter).

Die Wechselintervallszeit, d. h. die Verwendungszeit einer Wundauflage bis zum nächsten Verbandwechsel, soll wenigstens 24 h betragen, wobei Bestrebungen bestehen, die Wechselintervallszeit zu erhöhen, insbesondere auf 48 bis 72 h. Dies wäre aus betriebswirtschaftlichen Gesichtspunkten, aber auch aus Gründen einer durch häufige Verbandwechsel verursachten Störung der Wundheilung wünschenswert. Auf der anderen Seite besteht solchenfalls die Gefahr, dass die Wundkonditionierung hierunter leiden würde, weshalb man bislang die Wechselintervallszeit bei kritischen Wunden in der interaktiven Nasstherapie im Bereich von 24 h gehalten hat.

US 2008/0082059 A1 erwähnt eine in einen Unterdruckwundverband integrierte Wundauflage mit einem Saugkörper, für den in verschiedenen Listen eine Vielzahl von Stoffen und Stoffzusätzen genannt sind, insbesondere eine Mehrzahl von Trägerstoffen, eine Mehrzahl von Stoffzusätzen, darunter Polyhexamethylene biguanide und isotonische Kochsalzlösung. Eine konkrete Zusammensetzung des Saugkörpers und seiner Umhüllung ist nicht offenbart. WO 2010/024928A1 offenbart einen Verbund umfassend eine Filmschicht, eine Kollagenschicht und eine Hydrogelschicht mit Polyhexamethylenbiguanid. WO 2003/039602 A2 lehrt bei einem Material zur Aufnahme von Wundexudat antimikrobiell wirkende Gruppen kovalent an ein Trägerpolymer zu binden und hierdurch zu lokalisieren.

Der vorliegenden Erfindung liegt die Aufgabe zugrunde, eine Wundauflage der eingangs beschriebenen Art im Hinblick auf eine Optimierung der Wundkonditionierung, insbesondere im Hinblick auf eine möglicherweise zu verlängernde Wechselintervallszeit, zu verbessern.

Diese Aufgabe wird erfindungsgemäß mit einer Wundauflage mit den Merkmalen des Anspruchs 1 gelöst.

Bislang wurden Substanzen mit antimikrobieller Wirkung in Saug-/Spülkörpern von Wundauflagen nicht verwendet, da man die Wunde im Wesentlichen frei von derartigen Substanzen halten wollte, oder es wurde bewusst ein Antiseptikum in die Wunde gegeben. Mit der vorliegenden Erfindung wurde erkannt, dass jedoch im Betriebszustand kationische Substanzen mit antimikrobieller Wirkung durch negative Gruppen typischer superabsorbierender Materialien derart gebunden und damit gehalten werden, dass sie auch im flüssigkeitsaustauschenden Betrieb des Saug-/Spülkörpers einer erfindungsgemäßen Wundauflage im feuchten oder feuchtnassen Wundmilieu weitestgehend innerhalb des Saug-/Spülkörpers verbleiben, also nicht oder nur unwesentlich in die Wunde gelangen. Somit kann eine hervorragende Konditionierung des Saug-/Spülkörpers auch für Anwendungen über 24 h hinaus realisiert werden, was sich wiederum vorteilhaft auf die Konditionierung des Wundmilieus auswirkt, da ja im Flüssigkeitsaustausch im Betrieb des Saug-/Spülkörpers Flüssigkeit aus dem antimikrobiell optimal konditionierten Saug-/Spülkörper zur Wunde gelangt, jedoch im wesentlichen ohne dass hierbei septisch wirkende Substanzen oder Keime zurück zur Wunde gelangen. Es wird also die Rückkontamination weitestgehend verhindert.

Durch die auf der wundzugewandten Seite partiell und strukturiert aufgebrachte atraumatisch wirkende Beschichtung wird in unvorhergesehener Weise ein besserer Flüssigkeitsaustausch über die beschichtungsfreien Bereiche erreicht, weil durch die angrenzenden atraumatisch beschichteten Bereiche eine flächenmäßig durchgehende Anhaftung zwischen der Umhüllung der Wundauflage und dem Wundgewebe aufgrund einer Abstandshalterfunktion bei den atraumatisch beschichteten Bereichen verhindert wird. Dies führt zu einem insgesamt besseren Flüssigkeitsaustausch, der sich wiederum im Zusammenwirken mit der kationischen Substanz mit antimikrobieller Wirkung im Inneren des Saug-/Spülkörpers förderlich auf die Wundheilung auswirkt, da Wundsekrete/-exsudate mit Keimen in größerem Umfang in den Saug-/Spülkörper gelangen können und dort antimikrobiell behandelt werden. Der Begriff der partiell und strukturiert aufgebrachten Beschichtung impliziert, dass es sich hierbei nicht um eine flächenhaft durchgehende Beschichtung handelt, sondern um eine poröse Beschichtung, wie sie beispielsweise durch Punkte, Inseln, Linien, Streifen oder sonstige zusammenhängende oder nicht zusammenhängende Strukturen realisiert werden kann.

Vorzugsweise handelt es sich bei dem anionischen superabsorbierenden Material um ein Polymer, insbesondere um ein zumindest partiell quervernetztes Polymer, insbesondere auf Polyacrylatbasis. Gemäß einer weiteren Ausführungsform handelt es sich bei dem anionischen superabsorbierenden Material um ein Polysaccharid wie beispielsweise Stärke, oder um ein Polysaccharidderivat, beispielsweise um carboxyalkylierte Polysaccharide, wie in der WO-A-2008/037082 beschrieben.

Bei der kationischen Substanz mit antimikrobieller Wirkung kann es sich beispielsweise um Substanzen mit Amino oder Iminogruppen handeln, welche in einer Lösung mit einem pH-Wert von 4 bis 7,5 kationisch geladen vorliegen. Weiterhin kann es sich bei der kationischen Substanz um antimikrobiell wirksame Metallkationen handeln, insbesondere um Silberkationen, beispielsweise um einen Komplex von 1-Vinyl-2-Pyrrolidon mit Silber-Kationen. Besonders geeignete kationischen Substanzen mit antimikrobieller Wirkung sind Biguanid-Derivate wie Chlorhexidin oder Polybiguanide, wie Polyethylene Biguanid (PEB), Polytetramethylenbiguanid (PTMB) oder Polyethylenhexamethylenbiguanide (PEHMB). Eine besonders bevorzugtes Polybiguanid ist Polyhexamethylenbiguanid (PHMB, bzw. Polyhexanid). Weitere geeignete kationischen Substanzen mit antimikrobieller Wirkung sind Polyguanidine wie zum Beispiel Polyhexamethylenguanidine (PHMG), *N*-Octyl-1-[10-(4-Octyliminopyridin-1-yl)decyl]pyridin-4-imin (Octeneidin), quartäre Ammoniumverbindungen, wie zum Beispiel Benzalkoniumchlorid oder Cetylpyridiniumchlorid, Triazine wie zum Beispiel 1-(3-Chloroallyl)-3,5,7-Triaza-1-Azoniaadamantan-Chlorid oder die Ammoniumverbindung Taurolidin.

Die Konzentration des antiseptisch wirkenden Zusatzes in der wässrigen Lösung, die dann zur Aktivierung des trockenen Saug/Spülkörpers verwendet wird, beträgt vorzugsweise 0,06 - 0,20 Gew.-%, insbesondere 0,08 - 0,15 Gew.-%, insbesondere 0,10 - 0,15 Gew.-% (bezogen auf die wässrige Lösung vor dem Aktivieren des Saug/Spülkörpers).

Wie oben bereits ausgeführt, wird durch die Wahl der Substanz mit antimikrobieller Wirkung im Zusammenwirken mit dem superabsorbierenden Material erreicht, dass diese Substanz auch im Betrieb der Wundauflage weitgehend oder weitestgehend innerhalb des Saug-/Spülkörpers verbleibt. Dies kann anhand des Hemmhoftests (Englisch: zone of inhibition test) visuell geprüft bzw. verifiziert werden. Bei der Durchführung des nachfolgend im Einzelnen beschriebenen Hemmhoftests kann visuell überprüft werden, ob aus einem Testkörper keimtötende Substanzen in wesentlicher Menge austreten oder nicht. Treten sie aus, so töten oder behindern sie das Wachstum sogenannter koloniebildender Einheiten in einer Umgebung des Testkörpers, was visuell durch Inaugenscheinnahme des Testkörpers und seines Umfelds festgestellt werden kann. Lässt sich keine solche Umgebung, also kein Hemmhof (oder englisch: zone of inhibition) feststellen, so bedeutet dies, dass aus dem Testkörper keine das Koloniewachstum in nennenswertem Umfang beeinträchtigende Substanzen ausgetreten sind. Zur noch weitergehenden Charakterisierung des Gegenstands der vorliegenden Erfindung ist die Wundauflage weiter dadurch gekennzeichnet, dass bei Ausführung des Hemmhoftests ein Austreten von kationischer Substanz mit antimikrobieller Wirkung aus der Wundauflage visuell nicht feststellbar ist. Dies bedeutet, dass ein Hemmhof bei Ausführung des nachfolgend beschriebenen Tests visuell nicht feststellbar ist.

### Hemmhoftest

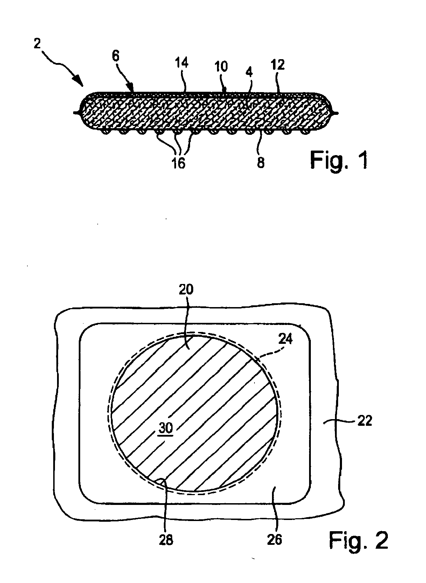

Zur Durchführung des Hemmhoftests wird eine Wundauflage der hier in Rede stehenden Art als Testkörper auf eine zuvor vorbereitete Agarplatte aufgelegt. Es wird hierfür eine Agarplatte mit einem Durchmesser von 8,5 cm eingesetzt, die jeweils 15 ml Caseinpepton-Sojapepton-Agar enthielten (Konzentration des Caseinpepton-Sojapepton: 40 g/I). Auf diese Agarplatte wurde 100 µl einer Staphylokokkus aureus ATCC 6538 Keimsuspension (etwa 5 x 10⁶ koloniebildende Einheiten/ml) mit einem Wattestäbchen aufgestrichen und für 10 bis 15 min. getrocknet. Nach dem Trocknen der Keimsuspension mit geschlossenem Deckel wird der sterilisierte, jedoch auf Raumtemperatur abgekühlte Testkörper in Form der zu testenden Wundauflage auf die Agarplatte mit der wundzugewandten Seite (Bezugszeichen 8 in Figur 1) aufgelegt. Die Platte wird dann aufrechtstehend für 18 h bei 35°C inkubiert. Danach wurde die Agarschale visuell nach der Bildung einer Hemmzone untersucht. Die Hemmzone wird typischerweise in Millimeter nach der Formel H = (D - d) : 2 berechnet, wobei D der Gesamtdurchmesser von Testkörper und Hemmzone und d der Durchmesser des Testkörpers jeweils in Millimeter ist. Sofern eine Hemmzone um den Testkörper herum visuell nicht feststellbar ist (D = d und H = 0), so ist der Austritt etwaiger Substanzen aus dem Testkörper und deren Auswirkung auf das Koloniewachstum nach dem Hemmhoftest nicht feststellbar.

Die Zusammensetzung des Saug-/Spülkörpers kann zweckmäßigerweise umfassen: ein superabsorbierendes Material der oben genannten Art in einer Menge von 120 - 170 g/m², cellulosische Fasern in einer Menge von 120 - 170 g/m² und insbesondere thermoplastische Fasern in einer Menge von 5 - 18 g/m². Zusätzlich kann auf einer oder beiden Seiten eine Tissueschicht vorgesehen sein. Der Saug-/Spülkörper kann aus den vorgenannten Komponenten (und zusätzlich der salzhaltigen wässrigen Lösung) bestehen.

Im Hinblick auf eine möglichst lange Wechselintervallszeit erweist es sich als wesentlich, dass hinreichend Flüssigkeit in dem Saug-/Spülkörper über die gesamte Dauer der Verwendung, also bis zum nächsten Verbandwechsel, zur Verfügung steht, damit die eingangs genannte duale Funktion des Saug-/Spülkörpers auch tatsächlich wirksam wird. Hierfür muss die Verdunstung der Flüssigkeit während der Anwendung der Wundauflage in akzeptablen Grenzen gehalten werden. Im Hinblick darauf erweist es sich als vorteilhaft, wenn die Umhüllung auf der wundabgewandten Seite einen Vliesstoff mit einer außenseitig aufkaschierten Folienschicht aufweist, welche verdunstungshemmend wirkt. Es hat sich gezeigt, dass hierdurch die Verdunstung im Vergleich zu vorbekannten Wundauflagen mit innerhalb der Umhüllung auf der wundabgewandten Seite des Saug-/Spülkörpers angeordneten oder eingelegten Wäscheschutzfolien erheblich reduziert werden kann.

Bei dem Vliesstoff handelt es sich in bevorzugter Weise um einen Polyolefin-Vliesstoff, insbesondere einen Polypropylen-Vliesstoff.

Die Umhüllung auf der wundzugewandten Seite der Wundauflage, aber grundsätzlich auch auf der wundabgewandten Seite der Wundauflage, kann in vorteilhafter Weise ein textiles Flächenmaterial, wie z.B. ein Gestrick, ein Gewirke oder Gewebe, insbesondere aus Polyolefin, insbesondere aus Polypropylen, sein. Jedenfalls ist die Verwendung einer Umhüllung in Form eines textilen Flächenmaterials, insbesondere Gestricks, Gewirks oder Gewebes, auf der wundzugewandten Seite im Hinblick auf den Flüssigkeitsaustausch, also im Hinblick auf die duale Funktion des Saug-/Spülkörpers, bevorzugt.

Die oben erwähnten vorteilhaften Gesichtspunkte der partiell und strukturiert aufgebrachten atraumatisch wirkenden Beschichtung, nämlich ein Verkleben mit Wundgewebe zu verhindern und eine gewisse Abstandshalterfunktion auszuüben, werden besonders gut durch eine Silikonbeschichtung erreicht. Wenn die atraumatisch wirkende Beschichtung in Form von Silikon auf ein textiles Flächenmaterial, wie vorausgehend erwähnt, aufgebracht wird, so ergibt sich hinsichtlich Flexibilität, Anschmiegsamkeit und Wirksamkeit ein besonders vorteilhafter Verbund.

Weiter erweist sich ein Bedeckungsgrad der partiell und strukturiert aufgebrachten atraumatisch wirkenden Beschichtung von 40 bis 70 %, insbesondere von 40 bis 60 % und weiter insbesondere von 40 - 55 Gew.-%, insbesondere 40 - 50 % der Fläche der betrachteten wundzugewandten Umhüllung als vorteilhaft.

Der Überstand der Beschichtung in Z-Richtung über die Ebene der Außenseite der Umhüllung beträgt vorzugsweise 0,03 - 0,5 mm, insbesondere 0,1 - 0,4 mm, 0,1 - 0,3 mm, insbesondere 0,1 -0,2 mm.

Die partiell und strukturiert aufgebrachte atraumatisch wirkende Beschichtung hat vorzugsweise eine Abmessung in wenigstens einer Ebenenrichtung von höchstens 4 mm, insbesondere von höchstens 3 mm, und weiter insbesondere von 1 - 3 mm, insbesondere von 2 - 3 mm.

Der Abstand benachbarter beschichteter Oberflächenbereiche voneinander beträgt vorzugsweise wenigstens 1 mm und höchstens 5 mm.

Im Hinblick auf die Ausbildung des faservliesbasierten Saug-/Spülkörpers erweist es sich als vorteilhaft, wenn zellulosische Fasern, vorzugsweise luftgelegte zellulosische Fasern oder vorzugsweise luftgelegte Mischungen aus zellulosischen Fasern und thermoplastischen Fasern, insbesondere Polyolefin-Fasern, insbesondere Polypropylen- oder Polypropylen/Polethylen-Fasern, verwendet werden, die vorzugsweise die gesamte Faserbasis des Saug-/Spülkörpers bilden. Der Anteil der thermoplastischen Fasern einer solchen Fasermischung beträgt vorzugsweise nur etwa 5 -10 % des Anteils zellulosischer Fasern.

Es erweist sich außerdem als vorteilhaft, wenn die flächenspezifische Verdunstungsrate im Zeitintervall 24h bis 72h nach der simulierten Aufbringung der Wundauflage < 0,020 g/24h·cm², insbesondere < 0,017 g/24h·cm², insbesondere < 0,015 g/24h·cm², insbesondere < 0,012 g/24h·cm² ist. Hierfür wird die nachfolgende Testmethode verwandt.

### Testmethode Verdunstungsrate:

Zur Bestimmung der Verdunstungsrate wird eine zunächst wasserdampfdicht verpackte Wundauflage 20 (Figur 2) aus ihrer Umverpackung entnommen. Sie weist einen faservliesbasierten Saug-/Spülkörper mit superabsorbierendem Material, insbesondere Favor pac 300 von der Firma Evonik Stockhausen GmbH, und typischerweise zellulosische Fasern und gegebenenfalls noch thermoplastische Fasern auf. Vor der wasserdampfdichten Verpackung wurde die Wundauflage 20 mit salzhaltiger wässriger Lösung, insbesondere Ringerlösung, aktiviert. Diese Wundauflage 20 wird sodann mit ihrer wundzugewandten Seite (Bezugszeichen 8 in Figur 1) auf eine wasserdampfundurchlässige Verpackungsfolie 22 (siehe Figur 2) gelegt, und ein umlaufender Randbereich 24 wird mit einer Folie 26 überfangen und so gegen die Unterlage fixiert (simulierte Aufbringung der Wundauflage). Hierfür wird eine Folie 26 (beispielsweise der Handelsmarke Opsite flexifix) über die Wundauflage 20 ausgebreitet, und es wird mittig, wie in Figur 2 skizziert, eine Öffnung 28 in die Abdeckfolie 26 geschnitten, so dass der größte Teil der Oberfläche 30 der Wundauflage 20 zur Umgebung exponiert ist und lediglich der umlaufende Randbereich 24 mit 5mm Breite durch die Abdeckfolie 26 überdeckt bleibt. Somit steht der größte Teil der körperabgewandten Seite der Wundauflage 20 zur Umgebung offen, so dass im Saug-/Spülkörper aufgenommene Flüssigkeit über diese Fläche verdampfen kann. Die betreffende Probe wird so bei 23° und 50% relativer Luftfeuchtigkeit gelagert und zu definierten Zeitpunkten, nämlich t = 0 h, t = 24 h, t = 48 h und t = 72 h gewogen. Durch Gewichtsdifferenzbetrachtung kann dann die Verdunstungsrate in g/24 h oder in g/24h·cm² angegeben werden. Bei der letzteren Größe handelt es sich um eine auf die Flächeneinheit der exponierten Fläche bezogene flächenspezifische Verdunstungsrate.

Es erweist sich ferner als vorteilhaft, wenn die flächenspezifische Verdunstung innerhalb der ersten 24 h nach der simulierten Aufbringung der Wundauflage < 0,050 g/24h·cm² Flüssigkeit, insbesondere < 0,037 g/24h·cm² Flüssigkeit, insbesondere < 0,025 g/24h·cm² Flüssigkeit ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Wundauflage. In der Zeichnung zeigt:
Figur 1 eine stark schematisierte Schnittansicht einer erfindungsgemäßen Wundauflage;
Figur 2 die Anordnung zur Durchführung des Tests zur Bestimmung der Verdunstungsrate.
Figur 3 zeigt das Ergebnis von Messungen.

Figur 1 zeigt im Schnitt eine Wundauflage 2. Sie umfasst einen Saug-/Spülkörper 4 auf Faservliesbasis. Bei dieser Faserbasis handelt es sich um eine bevorzugte Mischung aus luftgelegten Zellulosefasern (Zellstoff) und Polypropylen-Fasern oder Polypropylen/Polyethylen-Fasern. Dieser Fasermischung sind superabsorbierende Polymermaterialien (SAP) in Partikelform oder in Faserform möglichst homogen beigemischt, wobei der SAP-Anteil an der Gesamtmasse des Saug-/Spülkörpers vorzugsweise 40 - 50 Gew.-% beträgt. Die mittlere Korngröße der SAP-Partikel liegt beispielsweise bei 150 bis 850 µm (beispielsweise Polyacrylat der Marke Favor pac 300 von der Firma Evonik Stockhausen GmbH).

Der Saug-/Spülkörper 4 ist von einer die Außenseiten der Wundauflage bildenden Umhüllung 6 umgeben, die von einer wundzugewandten Hüllschicht 8 und einer wundabgewandten Hüllschicht 10 gebildet ist, die randseitig miteinander verbunden sind. Bei der wundzugewandten Hüllschicht 8 handelt es sich vorzugsweise um ein Gestrick, vorzugsweise aus Polypropylen, wobei auch ein Gewebe oder Gewirke in vorteilhafter Weise denkbar wäre, also eine Hüllschicht aus Fäden oder Filamenten mit textiler Bindung, die einen guten Flüssigkeitsaustausch zwischen Saug-/Spülkörper 4 und Wundumgebung gestattet.

Die wundabgewandte Hüllschicht 10 ist von einem Flächenverbund oder -laminat gebildet, und zwar von einem Vlies 12, vorzugsweise aus Polypropylen, und einer darauf aufkaschierten keim- und wasserdichten Folie 14, die sich demgemäß über die gesamte flächenhafte Erstreckung des Vlieses 12 erstreckt und zur wundabgewandten Seite hin einen wesentlich verbesserten Verdunstungsschutz bildet.

Auf der wundzugewandten Außenseite der wundzugewandten Hüllschicht 8 ist eine partiell und strukturiert aufgebrachte atraumatisch wirkende Beschichtung 16 vorgesehen. Diese Beschichtung ist vorzugsweise eine Silikonbeschichtung, wobei die Beschichtung porös und im beispielhaft dargestellten Fall von einer Mehrzahl von verhältnismäßig dünnen Streifen oder Linien oder inselförmigen Bereichen gebildet ist, die durch nicht beschichtete Bereiche voneinander separiert sind. In diesen nicht beschichteten Bereichen ist die wundzugewandte Hüllschicht 8 zur Wunde exponiert. Die atraumatisch wirkende Beschichtung 16 bildet diesbezüglich einen Überstand in der in der Beschreibungseinleitung genannten Größenordnung, wodurch einerseits ein Verkleben der Hüllschicht 8 mit Wundgewebe verhindert werden kann und andererseits ein gewisser geringer Abstand zwischen der wundzugewandten Hüllschicht und dem Wundgewebe beibehalten werden kann, wodurch das poröse Hüllschichtmaterial dreidimensional offen bleibt und einen geringeren Widerstand für den Flüssigkeitsdurchtritt in beiden Richtungen über die Gebrauchsdauer der Wundauflage bereitstellt oder behält.

Die Wundauflage 2 weist ferner einen in Umfangsrichtung erstreckten Rand auf, der durch die beiden dort miteinander verschweißten Hüllschichten 8, 10 gebildet ist. Die Wundauflagen werden jedoch so ausgestanzt, dass der Rand weitestgehend zu vernachlässigen ist.

Die vorstehend beschriebene atraumatisch wirkende Beschichtung 16 wird nicht im endlosen Flachmaterial der Hüllschichten, sondern erst nach dem letzten Schweißvorgang, insbesondere nach dem Vereinzeln der Wundauflage-Produkte aufgebracht, also nachdem die jeweiligen Saug-/Spülkörper 4 zwischen der wundzugewandten Hüllschicht 8 und der wundabgewandten Hüllschicht 10 durch Fügen der Hüllschichten 8, 10 miteinander fixiert wurden. Die so hergestellte Produktanordnung oder die bereits vereinzelten Produktstanzlinge werden dann mit Beschichtungsmaterial, vorzugsweise mit 1-Komponenten Silikon aus einer Dispensieranlage in der vorstehend beschriebenen Weise partiell und strukturiert beaufschlagt. Hierfür wird eine Dosiervorrichtung mit einer präzise ausmündenden nadelförmigen Dosierdüse verwendet, die ein gesteuert öffnendes und schließendes Ventil umfasst. Die Dosiervorrichtung ist in X- und Y-Richtung und vorzugsweise auch in Z-Richtung programmiert gesteuert verfahrbar. Auf diese Weise kann ein partieller und strukturierter Auftrag, insbesondere mit den eingangs angegebenen Parametern, zur Abmessung und zum Überstand der beschichteten Oberflächenbereiche erreicht werden.

Erst danach wird der Saug-/Spülkörper 4 der erfindungsgemäßen Wundauflage mit salzhaltiger wässriger Lösung, insbesondere Ringerlösung, vorzugsweise bis zur Sättigung beaufschlagt. Dieser salzhaltigen Lösung ist eine Substanz mit antimikrobieller Wirkung zugegeben, welche im feuchten oder feuchtnassen Wundmilieu bei pH-Werten im leicht sauren bis neutralen Bereich von pH 4 bis 7,5 kationisch vorliegt. Diese kationische Substanz mit antimikrobieller Wirkung wird von negativen Gruppen des anionischen superabsorbierenden Materials derart angezogen, dass sie auch im flüssigkeitsaustauschenden Betrieb des Saug-/Spülkörpers 4 an die superabsorbierenden Materialien gebunden bleibt, also weitestgehend nicht in das Wundmilieu abgegeben wird. Hierdurch werden mit Wundsekret in den Saug-/Spülkörper 4 eingebrachte Keime an einer Vermehrung gehindert, wodurch eine Rückkontamination in Richtung auf die Wunde weitestgehend verhindert wird. Es wurde festgestellt, dass so eine mikrobiologische Rückkontamination über 72 h weitestgehend verhindert werden konnte, was darauf zurückgeführt wird, dass die antimikrobiell wirkende Substanz aufgrund ihres kationischen Zustands gleichmäßig verteilt innerhalb des Saug-/Spülkörpers 4 in Anbindung an die superabsorbierenden Materialien gehalten wird.

Bei einer beispielhaften bevorzugten Zusammensetzung der Wundauflage 2 besteht die Faservliesbasis des Saug-/Spülkörpers 4 aus 127 g/m² Zellulosefasern (Zellstoff), 8 g/m² Polypropylen/Polyethylen-Fasern als Bindefasern (insbesondere E-505/FV von Firma Schwartswalder). Dieser Fasermischung sind 127 g/m² der oben genannten superabsorbierenden Polymermaterialien (SAP) homogen beigemischt. Die so erhaltene Mischung (Faservliesbasis + SAP), welche den Saug/Spülkörper 4 bildet, kann zudem von einer zellulosischen Tissueschicht, insbesondere eines Flächengewichts von beispielhaft 18 g/m² auf jeder Seite, umgeben sein (Diaper Tissue 1800 von Swedish Tissue AB) (in der Figur nicht dargestellt); dies ist jedoch nicht zwingend erforderlich. Die Wundauflage kann beispielhaft rund ausgebildet sein, mit einer beispielhaften Abmessung des Saug/Spülkörpers 4, die im wesentlichen der Abmessung der Wundauflage 2 entspricht, von 5,5 cm Durchmesser. Die Wundauflage 2 wurde schließlich mit 13,6 ml Ringerlösung aktiviert, was vorliegend im wesentlichen einer Sättigung des Saug-/Spülkörpers mit Flüssigkeit entspricht. Die Umhüllung 6 ist wie vorausgehend beschrieben ausgebildet. - Bei einer derartigen Wundauflage wurde eine Verdunstungsrate während der ersten 24 h nach der simulierten Aufbringung von 0,19 g/24h ermittelt. Hier wäre eine vorteilhafte Obergrenze 0,8 g/24h, insbesondere 0,6 g/24h, insbesondere 0,4 g/24h. - Während des nachfolgenden Zeitintervalls von 24 h bis 72 h betrug die Verdunstung 0,16 g/24h. Hier wäre eine vorteilhafte Obergrenze 0,3 g/24h, insbesondere 0,2 g/24h.

### Trennkraftmessung:

Es wurde schon darauf hingewiesen, dass die Ablösbarkeit oder Entfernbarkeit der Wundauflage von der Wunde bei einem Verbandwechsel eine sehr wesentliche Eigenschaft darstellt, da eine zu starke Anhaftung oder gar Verkleben mit dem Wundgrund zu Schmerzen beim Ablösen und auch zu einer erneuten Schädigung des Wundgrunds führt. Die nachfolgend beschriebene Trennkraft-Messung soll zur Bestimmung der Neigung zum Verkleben mit dem Wundgrund herangezogen werden und somit eine Beurteilung für die Ablösbarkeit oder Entfernbarkeit einer Wundauflage von der Wunde zulassen:

Als Mass für die Ablösbarkeit oder Entfernbarkeit dient die Messung der Trennkraft zwischen einem textilen Substrat und Gelatine. Bei dem textilen Substrat handelt es sich um die wundberührende textile Oberflächenschicht der Wundauflage, und die Gelatine soll das Wundmedium bzw. die Hautoberfläche simulieren.

Es wird eine wässrige, 20%ige Gelatine-Lösung hergestellt (ca. 1.5 h bei 60°C halten um die Gelatine zu lösen); es wird davon ca. 100 ml in eine Petrischale (mit Abmessungen 12cmx12 cm) gegossen. Nach 30 min Abkühlzeit werden jeweils die zu untersuchenden Proben (Grösse 2.5cmx10 cm) auf die Gelatine in der Petrischale aufgelegt und mit Gewichten (20mmx90 mm, 4.2 g) beschwert. Anschließend erfolgt die Inkubation der Gelatineplatten für 3 h bei 36°C und 50% rel. Feuchte. Vor der Kraftmessung werden die Gelatineplatten für 1 h bei 23°C und 50% rel. Feuchte klimatisiert.

Zur Messung der Trennkraft wird ein jeweiliges Probenende leicht aus dem Gelatinebett abgelöst und mit einem Klebeband verlängert. Die Petrischale und das Ende der Klebebänder wird in die Halterungen der Zugprüfmaschine eingespannt und dann die Trennkraft gemessen. Der Abzug der Zugprüfmaschine erfolgt ungefähr vertikal, während die jeweilige Gelatineplatte/Petrischale horizontal angeordnet ist. Ausgewertet wird die mittlere Trennkraft über den Messweg.

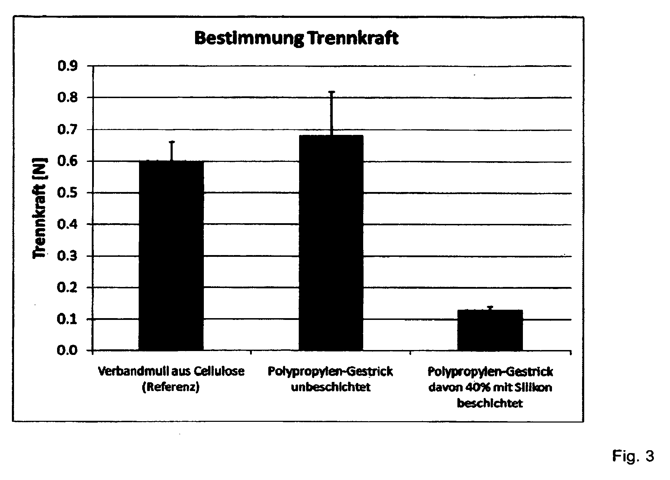

Figur 3 zeigt das Ergebnis von Messungen an folgenden Proben: Die Trennkraft wurde zum einen an einem Polypropylen-Gestrick, dessen Fläche zu 40 % mit Silikon beschichtet war, bestimmt. Das Silikon war punktförmig aufgetragen.

Nicht beschichtetes Polypropylen-Gestrick und Verbandmull wurden als Vergleichsproben benutzt.

Man erkennt somit, dass das mit Silikon beschichtete Polypropylen-Gestrick wesentlich besser als Umhüllung des Saug-/Spülkörpers auf der wundzugewandten Seite geeignet ist als Verbandmull oder unbeschichtetes Polypropylen-Gestrick.

### Anwendungsbeobachtung

Die Anmelderin führte zu ihrer erfindungsgemäßen Wundauflage eine klinische Anwendungsbeobachtung (AWB) durch. Die Wundauflage hatte die oben beschriebene Zusammensetzung mit einer atraumatisch wirkenden Beschichtung in Form von Silikon in punktiert strukturiertem Auftrag mit einem Bedeckungsgrad von 40 % der wundzugewandten Fläche der Hüllschicht 8. Die salzhaltige wässrige Lösung des Saug-/Spülkörpers 4 war Ringerlösung mit 0,1 Gew.-% PHMB als kationischer Substanz mit antimikrobieller Wirkung.

Die Anwendungsbeobachtung umfasste den Zeitraum der Eingangsuntersuchung E mit einer ersten Applikation der Wundauflage bis zur Abschlussuntersuchung A mit einem Wechsel der Wundauflage. Im dazwischen liegenden Zeitraum wurden zwei weitere Verbandwechsel durchgeführt.

Die Eingangsuntersuchung wurde bei 66 Patienten durchgeführt. Pro Patient war die Dokumentation von drei aufeinander folgenden Verbandwechseln geplant. 3 Patienten standen zum Zeitpunkt des ersten Verbandwechsels nicht mehr für die Anwendungsbeobachtung zur Verfügung. 4 weitere Patienten standen zum Zeitpunkt des zweiten Verbandwechsels nicht mehr für die Anwendungsbeobachtung zur Verfügung. Die Abschlussuntersuchung erfolgte an 59 Patienten. Wenn möglich, wurde jedoch versucht, trotzdem eine abschließende Produktbeurteilung zu erhalten und diese dann auch in die Auswertung einzubeziehen. Für die abschließende Produktbeurteilung standen daher Daten von 63 Patienten zur Verfügung.

Je 50% der Patienten (33) waren männlich bzw. weiblich.

Das Durchschnittsalter aller Patienten lag bei etwa 75 Jahren.Das durchschnittliche Gewicht der Patienten betrug etwa 77 kg, Minimum 43,5 kg, Maximum 122,0 kg, Median 78 kg. Die durchschnittliche Größe lag bei 1,69 m, Minimum 1,48 m, Maximum 1,92 m, Median 1,68 m.

Die Wundursachen verteilten sich wie folgt:

| **Ursache** | **Anzahl (n)** | **Anteil (%)** |
|---|---|---|
| Ulcus cruris venosum | 10 | 15,2 |
| Ulcus cruris arteriosum | 6 | 9,1 |
| Ulcus cruris mixtum | 10 | 15,2 |
| Dekubitus | 19 | 28,8 |
| Druckulcus bei Diabetes | 9 | 13,6 |
| diabetisches Gangrän | 4 | 6,1 |
| traumatische Wunde | 3 | 4,5 |
| Sonstiges | 5 | 7,6 |

Die Wunden waren bei Behandlungsbeginn im Durchschnitt 5,26 ± 3,08 cm lang (Minimum 0,5 cm, Maximum 16,0 cm, Median 5,0 cm) und 3,94 ± 2,08 cm breit (Minimum 0,3 cm, Maximum 10,0 cm, Median 4,0 cm). In einem Fall wurde keine Wundgröße angegeben.

Bei 58 Wunden lagen außerdem Angaben zur Wundtiefe vor. Die durchschnittliche Wundtiefe betrug 0,93 ± 0,1 cm (Minimum 0,1 cm, Maximum 5,5 cm, Median 0,5 cm).

Bei den restlichen Wunden handelt es sich um oberflächliche Wunden.

Zu Beginn der AWB bestanden die Wunden im Durchschnitt seit 1,3 ± 3,5 Jahren (Minimum 0 Tag, Maximum 20,0 Jahre, Median 4,0 Monate). Bei 7 Patienten fehlen die Angaben zum Wundalter.

6 Wunden (9,1%) exsudierten nicht, 19 (28,8%) wenig, 28 (42,4%) mäßig, 12 (18,2%) stark und 1 (1,5%) sehr stark.

12 Wunden (18,2%) wiesen zu Beginn der Anwendungsbeobachtung Infektionszeichen auf.

Zeiten zwischen den Verbandwechseln: In der folgenden Tabelle sind die Zeiten zwischen den Verbandwechseln sowie die Gesamtdauer der Anwendungsbeobachtung in Tagen zusammengestellt. Dabei wurden die Patienten nicht berücksichtigt, bei denen aufgrund der Zeit zwischen den dokumentierten Verbandwechseln ziemlich sicher davon auszugehen ist, dass es sich nicht um aufeinander folgende Verbandwechsel gehandelt hat.

| | **E bis 1.VW** | **1.VW bis 2.VW** | **2.VW bis A** | **E bis A alle*** | **E bis A 3 VW*** |
|---|---|---|---|---|---|
| **Durch-schnitt** | 2,59 ±0,93 | 2,64 ±0,94 | 2,71 ±0,87 | 7,48±2,45 | 7,80 ± 2,36 |
| **Minimum** | 1 | 1 | 1 | 3 | 3 |
| **Maximum** | 6 | 6 | 5 | 12 | 12 |
| **Median** | 3 | 3 | 3 | 8 | 9 |

| | | | | | |
|---|---|---|---|---|---|
| E = Eingangsuntersuchung, VW = Verbandwechsel, A = Abschlussuntersuchung E = Eingangsuntersuchung, VW = Verbandwechsel, A = Abschlussuntersuchung * E bis A alle: hier wurden auch die Patienten einbezogen, bei denen die AWB bereits nach dem ersten oder zweiten Verbandwechsel vorzeitig beendet wurde. ** E bis A 3 VW: hier wurden nur die Patienten berücksichtigt, die die AWB planmäßig nach dem 3. Verbandwechsel beendet haben | | | | | |

### Schmerzen beim Verbandwechsel

In der folgenden Tabelle ist die Zahl der Patienten mit Schmerzen beim Verbandwechsel dargestellt

| | **Eingangsuntersuchung** | | **1. Verbandwechsel** | | **2. Verbandwechsel** | | **AbschlussUntersuchung** | |
|---|---|---|---|---|---|---|---|---|
| | **Anzahl** | **%** | **Anzahl** | **%** | **Anzahl** | **%** | **Anzahl** | **%** |
| Keine Schmerzen bei VW | 31 | 47,0 | 36 | 57,1 | 36 | 61,0 | 36 | 57,1 |
| Leichte Schmerzen bei VW | 15 | 22,7 | 17 | 27,0 | 15 | 25,4 | 21 | 33,3 |
| Mäßige Schmerzen bei VW | 16 | 24,2 | 7 | 11,1 | 6 | 10,1 | 5 | 7,9 |
| Starke Schmerzen bei VW | 4 | 6,1 | 3 | 4,8 | 2 | 3,4 | 1 | 1,6 |

Man erkennt eine signifikante Verbesserung des Schmerzempfindens bei Verbandwechsel zwischen dem Zeitpunkt der Eingangsuntersuchung und dem Zeitpunkt der Abschlussuntersuchung, die auch auf die erfindungsgemäß verbesserte Ablösbarkeit/Entfernbarkeit der verwandten Wundauflage von der Wunde zurückgeführt wird, die ihrerseits auf ein Zusammenwirken der Komponenten der Wundauflage zurückgeführt wird und die Wundheilung insgesamt positiv unterstützt.

### Entfernbarkeit der Wundauflage

Die Wundauflage ließ sich in 95,3 % der Fälle "sehr gut" (48,4 %) oder "gut" (46,9 %) von den Wunden entfernen. Da die Frage der Entfernbarkeit von großer Bedeutung ist und diese eventuell auch von der Tragedauer des Wundkissens beeinflusst wird, wurde die Entfernbarkeit auch nach jedem einzelnen Verbandwechsel untersucht. Dabei kam es beim Entfernen des Wundkissens in keinem Falle zu Problemen. Es konnte jedoch beobachtet werden, dass die Wundkissen bei längerer Tragedauer (ein, zwei oder drei Tage) öfter leicht auf den Wunden festhafteten (3,8 % - 9,1 % - 17,5 %). Sie konnten aber trotzdem immer problemlos entfernt werden. Auch nach einer Tragedauer von drei Tagen war in 82,5 % der Fälle kein Verkleben zu beobachten.

Diese Ergebnisse lassen den Schluss zu, dass die Wundauflage auch nach einer Tragedauer von drei Tagen nicht mit der Wunde verklebt.

### Feuchtigkeit des Wundkissens

In 54 von 63 Fällen (85,7%) war das Wundkissen beim Verbandwechsel noch optimal feucht. In 6 Fällen (9,5%) war das Wundkissen zwar noch ausreichend feucht, die Anwender hätten jedoch mehr erwartet. 3 mal (4,8%) empfanden die Anwender die Feuchtigkeit als nicht mehr optimal, ein deutlich zu trockenes Wundkissen wurde in keinem Fall beobachtet.

### Infektionsanzeichen

7 von 63 Wunden (11,1%) zu denen zu diesem Punkt Informationen vorlagen, wiesen zum Zeitpunkt der Abschlussbeurteilung Zeichen einer Wundinfektion auf.

### Drapierfähigkeit

Unter der Drapierfähigkeit verstehet man die Anpassungsfähigkeit der Wundauflage an den Wundgrund; so soll insbesondere verhindert werden, dass sich die Wundauflage abhebt. Die Drapierfähigkeit der verwandten Wundauflage wurde bei Beginn der AWB 14 mal als sehr gut (21,2%), 44 mal als gut (66,7%), 7 mal als befriedigend (10,1%), 1 mal als ausreichend (1,5%) und kein mal als mangelhaft beurteilt.

### Wundzustand im Vergleich zum Studienbeginn

Bei 62 Patienten lag eine Beurteilung der Wunde im Vergleich zum Studienbeginn vor. Dabei ergab sich folgendes Bild

| **Zustand** | **Anzahl** | **Anteil (%)** |
|---|---|---|
| Deutlich verbessert | 15 | 24,2 |
| Verbessert | 34 | 54,8 |
| Unverändert | 7 | 11,3 |
| verschlechtert | 1 | 1,6 |
| Deutlich verschlechtert | 5 | 8,1 |

### Gesamteindruck

Bei der AWB wurde der Gesamteindruck der Wundauflage in 87,3 % aller Fälle mit "sehr gut" oder "gut" bewertet. Dieser positive Eindruck wurde auch dadurch bestätigt, dass die Erwartungen, die an das neue Produkt gestellt wurden, in 87,5 % der Fälle "übertroffen", "erfüllt" oder "überwiegend erfüllt wurden.

## Patentansprüche

1. Wundauflage (2) zur Wundbehandlung im feuchten oder feuchtnassen Milieu, mit einem faservliesbasierten Saug-/Spülkörper (4), in dem superabsorbierendes Material verteilt aufgenommen ist, wobei der Saug-/Spülkörper (4) herstellerseitig mit einer salzhaltigen wässrigen Lösung, insbesondere Ringerlösung, vorzugsweise bis zur Sättigung, beaufschlagt ist, und mit einer die äußeren Sichtseiten der Wundauflage bildenden Umhüllung (6), **dadurch gekennzeichnet, dass** das superabsorbierende Material anionisch ist und negative Gruppen aufweist und dass die wässrige Lösung eine Substanz mit antimikrobieller Wirkung umfasst, bei der es sich um Silberkationen, Biguanid oder Biguanid-Derivate, Polyguanidine, *N*-Octyl-1-[10-(4-Octyliminopyridin-1-yl)decyl]pyridin-4-imin (Octenidin), quartäre Ammoniumverbindungen, Triazine oder die Ammoniumverbindung Taurolidin handelt, und dass diese Substanz mit antimikrobieller Wirkung bei pH-Werten von 4 - 7,5 eines typischen feuchten oder feuchtnassen Wundmilieus kationisch geladen vorliegt-und daher von negativen Gruppen des anionischen superabsorbierenden Materials attraktiv angezogen wird und so innerhalb des Saug-/Spülkörpers (4) antimikrobiell wirkt, und dass die Umhüllung (6) auf der wundzugewandten Seite eine außenseitig partiell und strukturiert aufgebrachte atraumatisch wirkende Beschichtung (16) mit einem Bedeckungsgrad von höchstens 70 % aufweist und dass die partiell und strukturiert aufgebrachte atraumatisch wirkende Beschichtung (16) eine Silikonbeschichtung ist.

2. Wundauflage nach Anspruch 1, **dadurch gekennzeichnet, dass** die kationische Substanz mit antimikrobieller Wirkung Polyhexamethylenbiguanid und dessen Derivate umfasst.

3. Wundauflage nach Anspruch 1 oder 2, **dadurch gekennzeichnet, dass** die Konzentration der kationischen Substanz mit antimikrobieller Wirkung in der wässrigen Lösung 0,06 - 0,20 Gew.-%, insbesondere 0,08 - 0,15 Gew.-%, insbesondere 0,10 - 0,15 Gew.-% beträgt.

4. Wundauflage nach Anspruch 1, 2 oder 3, **dadurch gekennzeichnet, dass** bei Ausführung des Hemmhoftests ein Austreten von kationischer Substanz mit antimikrobieller Wirkung aus der Wundauflage visuell nicht feststellbar ist.

5. Wundauflage nach einem oder mehreren der vorstehenden Ansprüche, **dadurch gekennzeichnet, dass** die Umhüllung (6) auf der wundabgewandten Seite einen Vliesstoff (12) mit einer außenseitig aufkaschierten Folienschicht (14) aufweist, welche eine verdunstungshemmende Folienschicht bildet.

6. Wundauflage nach Anspruch 5, **dadurch gekennzeichnet, dass** der Vliesstoff (12) ein Polyolefin-Vliesstoff, insbesondere ein Polypropylen-Vliesstoff ist.

7. Wundauflage nach einem oder mehreren der vorstehenden Ansprüche, **dadurch gekennzeichnet, dass** die Umhüllung (6) auf der wundzugewandten und/oder auf der wundabgewandten Seite der Wundauflage von einem textilen Flächenmaterial, insbesondere von einem Gestrick, Gewirke oder Gewebe, insbesondere aus Polyolefin, insbesondere aus Polypropylen, gebildet ist.

8. Wundauflage nach einem oder mehreren der vorstehenden Ansprüche, **dadurch gekennzeichnet, dass** der Bedeckungsgrad der partiell und strukturiert aufgebrachten atraumatisch wirkenden Beschichtung (16) 40 - 70 %, insbesondere 40 - 60 %, insbesondere 40 - 55 Gew.-%, insbesondere 40 - 50 % beträgt.

9. Wundauflage nach einem oder mehreren der vorstehenden Ansprüche, **dadurch gekennzeichnet, dass** die partiell und strukturiert aufgebrachte atraumatisch wirkende Beschichtung (16) eine Abmessung in wenigstens einer Ebenenrichtung von höchstens 4 mm, insbesondere von höchstens 3 mm, und weiter insbesondere von 1 - 3 mm, insbesondere von 2 - 3 mm, aufweist.

10. Wundauflage nach einem oder mehreren der vorstehenden Ansprüche, **dadurch gekennzeichnet, dass** der Abstand benachbarter beschichteter Oberflächenbereiche voneinander wenigstens 1 mm und höchstens 5 mm beträgt.

11. Wundauflage nach einem oder mehreren der vorstehenden Ansprüche, **dadurch gekennzeichnet, dass** der faservliesbasierte Saug-/Spülkörper (4) cellulosische Fasern, insbesondere eine Mischung aus cellulosischen Fasern und thermoplastischen Fasern, insbesondere Polyolefin-Fasern, insbesondere Polypropylen-Fasern oder Polypropylen/Polyethylen-Fasern, umfasst.

12. Wundauflage nach einem oder mehreren der vorstehenden Ansprüche, **dadurch gekennzeichnet, dass** die flächenspezifische Verdunstungsrate im Zeitintervall 24h bis 72h nach der simulierten Aufbringung der Wundauflage < 0,020 g/24h·cm², insbesondere < 0,017 g/24h·cm², insbesondere < 0,015 g/24h·cm², insbesondere < 0,012 g/24h·cm² beträgt, bestimmt gemäß der in der Beschreibung beschriebenen Testmethode Verdunstungsrate.

13. Wundauflage nach einem oder mehreren der vorstehenden Ansprüche, **dadurch gekennzeichnet, dass** die flächenspezifische Verdunstung innerhalb der ersten 24 h nach der simulierten Aufbringung der Wundauflage < 0,050 g/24h·cm² Flüssigkeit, insbesondere < 0,037 g/24h·cm² Flüssigkeit, insbesondere < 0,025 g/24h·cm² Flüssigkeit beträgt, bestimmt gemäß der in der Beschreibung beschriebenen Testmethode Verdunstungsrate.

14. Wundauflage nach einem oder mehreren der vorstehenden Ansprüche, **gekennzeichnet durch** eine Abmessung in wenigstens einer Ebenenrichtung von 2 - 30 cm, insbesondere von 3 - 20 cm, insbesondere von 3 - 15 cm.

## Claims

1. Wound dressing (2) for wound treatment in the moist or wet milieu, with a fiber non woven based suction-/rinsing body (4), in which superabsorbent material is distributed, wherein the suction-/rinsing body (4) is supplied by the manufacturer with a saline aqueous solution, in particular Ringer's solution, preferably to the point of saturation, and with a cover (6) which forms the outer visible sides of the wound dressing, **characterized in that** the superabsorbent material is anionic and comprises negative groups and **in that** the aqueous solution includes an antimicrobially acting substance which is formed by silver cations, biguanide oder biguanide derivates, polyguanidine, *N*-Octyl-1-[10-(4-Octyliminopyridine-1-yl)decyl]pyridine-4-imine (Octenedine), quarternary amino compounds, triazines or the ammonia compound taurolidine, and **in that** this antimicrobially acting substance is cationic at pH values of 4 - 7.5 of a typical moist or wet wound milieu, and is therefor attracted by negative groups of the anionic superabsorbent material and in this way acts antimicrobially inside the suction-/rinsing body (4), and **in that** the cover (6) has an atraumatically acting coating which is applied in regions and in a structured manner on the outside of the side of the cover which faces toward the wound, with a degree of coverage of at most 70% and **in that** the atraumatically acting coating (16) which is applied in regions and in a structured manner, is a silicone coating.

2. Wound dressing according to claim 1, **characterized in that** the antimicrobially acting cationic substance comprises polyhexamethylenbiguanide and its derivates.

3. Wound dressing according to claim 1 or 2, **characterized in that** the concentration of the antimicrobially acting cationic substance in the aqueous solution is 0.06 -0.20 weight %, in particular 0.08 - 0.15 weight %, in particular 0.10 - 0.15 weight %.

4. Wound dressing according to claim 1, 2 or 3, **characterized in that** when performing the zone of inhibition test a release of antimicrobially acting cationic substance from the wound dressing is visually undetectable.

5. Wound dressing according to one or multiple of the preceding claims, **characterized in that** the cover (6) has a non woven fabric (12) on the side which faces away from the wound with a film layer (14) which is laminated on the outside and which forms an evaporation inhibiting film layer.

6. Wound dressing according to claim 5, **characterized in that** the non woven fabric (12) is a polyolefin non woven fabric, in particular a polypropylene non woven fabric.

7. Wound dressing according to one or more of the preceding claims, **characterized in that** the cover (6) on the side of the wound dressing which faces toward the wound and/or on the side of the wound dressing which faces away from the wound is formed by a textile flat material, in particular a knitted fabric, crocheted fabric or woven fabric, in particular made of polyolefin, in particular of polypropylene.

8. Wound dressing according to one or more of the preceding claims, **characterized in that** the coverage proportion of the atraumatically acting coating (16) which is applied in regions and in a structured manner, is 40 - 70%, in particular 40 - 60%, in particular 40 - 55 weight%, in particular 40 - 50%.

9. Wound dressing according to one or more of the preceding claims, **characterized in that** that atraumatically acting coating (16) which is applied in regions and in a structured manner has a dimension in at least one plane direction of at most 4 mm, in particular of at most 3 mm, and further in particular of 1 - 3 mm, in particular of 2 - 3 mm.

10. Wound dressing according to one or more of the preceding claims, **characterized in that** that the distance of neighboring coated surface areas to each other is at least 1 mm and at most 5 mm.

11. Wound dressing according to one or more of the preceding claims, **characterized in that** that the fibrous non woven based suction-/rinsing body (4) includes cellulosic fibers, in particular a mixture of cellulosic fibres and thermoplastic fibers, in particular polyolefine-fibers, in particular polypropylene-fibers or polypropylene/polyethylene-fibers.

12. Wound dressing according to one or more of the preceding claims, **characterized in that** that the surface specific evaporation rate in the time interval 24 h to 72 h after the simulated placement of the wound dressing is < 0.020 g/24 h cm², in particular < 0.017 g/24 h cm², in particular < 0 .015 g/24 h cm², in particular 0.012 g/24 h cm², and is obtained according to the test method evaporation rate explained in the description.

13. Wound dressing according to one or more of the preceding claims, **characterized in that** that the surface specific evaporation within the first 24 h after the simulated placement of the wound dressing is < 0.050 g/24 h cm² liquid, in particular < 0.037 g/24 h cm² liquid, in particular < 0.025 g/24 h cm² liquid, and is obtained according to the test method evaporation rate explained in the description.

14. Wound dressing according to one or more of the preceding claims, **characterized by** a dimension in at least one plane direction of 2 - 30 cm, in particular of 3- 20 cm, in particular of 3 - 15 cm.

## Revendications

1. Pansement pour plaie (2) destiné au traitement de plaies en milieu humide ou humide-mouillé, comprenant un corps d'absorption/de rinçage (4) à base de non-tissé fibreux dans lequel du matériau superabsorbant est distribué, ledit corps d'absorption/de rinçage (4) étant alimenté, chez le fabricant, en une solution aqueuse saline, en particulier une solution de Ringer, de préférence jusqu'à saturation, et comprenant une enveloppe (6) formant les faces visibles extérieures du pansement pour plaie, **caractérisé par le fait que** le matériau superabsorbant est anionique et présente des groupes négatifs et que ladite solution aqueuse comprend une substance à action antimicrobienne dans le cas de laquelle il s'agit de cations d'argent, de biguanide ou de dérivés de biguanide, de polyguanidines, de *N*-octyl-1-[10-(4-octyliminopyridine-1-yl)décyl]pyridine-4-imine (octénidine), de composés d'ammonium quaternaires, de triazines ou du composé d'ammonium dit taurolidine, et que, à des valeurs pH comprises entre 4 et 7,5 d'un milieu de plaie humide ou humide-mouillé typique, cette substance à action antimicrobienne est chargée cationiquement et est, pour cette raison, attirée de manière attrayante par des groupes négatifs du matériau superabsorbant anionique et agit ainsi de façon antimicrobienne à l'intérieur dudit corps d'absorption/de rinçage (4), et que ladite enveloppe (6) présente, sur la face montrant vers la plaie, un revêtement (16) à action atraumatique appliqué de façon partielle et structurée sur la face extérieure et ayant un taux de recouvrement d'au plus 70 %, et que ledit revêtement (16) à action atraumatique appliqué de façon partielle et structurée est un revêtement de silicone.

2. Pansement pour plaie selon la revendication 1, **caractérisé par le fait que** la substance cationique à action antimicrobienne comprend du biguanide de polyhexaméthylène et les dérivés de celui-ci.

3. Pansement pour plaie selon la revendication 1 ou 2, **caractérisé par le fait que** la concentration de la substance cationique à action antimicrobienne dans la solution aqueuse est comprise entre 0,06 et 0,20 % en poids, en particulier entre 0,08 et 0,15 % en poids, en particulier entre 0,10 et 0,15 % en poids.

4. Pansement pour plaie selon la revendication 1, 2 ou 3, **caractérisé par le fait que**, lorsqu'on effectue l'épreuve de diffusion en gélose, on ne peut pas constater visuellement que de la substance cationique à action antimicrobienne sort du pansement pour plaie.

5. Pansement pour plaie selon l'une ou plusieurs des revendications précédentes, **caractérisé par le fait que**, sur la face opposée à la plaie, ladite enveloppe (6) présente un non-tissé (12) ayant une couche de film (14) qui est laminée sur la face extérieure et qui forme une couche de film anti-évaporation.

6. Pansement pour plaie selon la revendication 5, **caractérisé par le fait que** ledit non-tissé (12) est un non-tissé en polyoléfine, en particulier un non-tissé en polypropylène.

7. Pansement pour plaie selon l'une ou plusieurs des revendications précédentes, **caractérisé par le fait que**, sur la face montrant vers la plaie et/ou sur la face opposée à la plaie, ladite enveloppe (6) est formée par un matériau textile en nappe, en particulier par un tricot ou un tissu, en particulier en polyoléfine, en particulier en polypropylène.

8. Pansement pour plaie selon l'une ou plusieurs des revendications précédentes, **caractérisé par le fait que** le taux de recouvrement dudit revêtement (16) à action atraumatique appliqué de façon partielle et structurée est compris entre 40 et 70 %, en particulier entre 40 et 60 %, en particulier entre 40 et 55 % en poids, en particulier entre 40 et 50 %.

9. Pansement pour plaie selon l'une ou plusieurs des revendications précédentes, **caractérisé par le fait que** le revêtement (16) à action atraumatique appliqué de façon partielle et structurée présente une dimension dans au moins une direction de plan qui est de 4 mm au plus, en particulier de 3 mm au plus, et encore en particulier comprise entre 1 et 3 mm, en particulier entre 2 et 3 mm.

10. Pansement pour plaie selon l'une ou plusieurs des revendications précédentes, **caractérisé par le fait que** la distance séparant des zones de surface revêtues voisines les unes des autres est de 1 mm au moins et de 5 mm au plus.

11. Pansement pour plaie selon l'une ou plusieurs des revendications précédentes, **caractérisé par le fait que** ledit corps d'absorption/de rinçage (4) à base de non-tissé fibreux comprend des fibres cellulosiques, en particulier un mélange de fibres cellulosiques et de fibres thermoplastiques, en particulier des fibres de polyoléfine, en particulier des fibres de polypropylène ou des fibres de polypropylène/polyéthylène.

12. Pansement pour plaie selon l'une ou plusieurs des revendications précédentes, **caractérisé par le fait que** le taux d'évaporation spécifique à la surface, dans l'intervalle de temps de 24 h à 72 h après l'application simulée du pansement pour plaie, est < 0,020 g/24 h·cm², en particulier < 0,017 g/24 h·cm², en particulier < 0,015 g/24 h·cm², en particulier < 0,012 g/24 h·cm², déterminé selon la méthode de test de taux d'évaporation qui est décrite dans la description.

13. Pansement pour plaie selon l'une ou plusieurs des revendications précédentes, **caractérisé par le fait que** l'évaporation spécifique à la surface, dans les premières 24 h après l'application simulée du pansement pour plaie, est < 0,050 g/24 h·cm² de liquide, en particulier < 0,037 g/24 h·cm² de liquide, en particulier < 0,025 g/24 h·cm² de liquide, déterminée selon la méthode de test de taux d'évaporation qui est décrite dans la description.

14. Pansement pour plaie selon l'une ou plusieurs des revendications précédentes, **caractérisé par** une dimension dans au moins une direction de plan, comprise entre 2 et 30 cm, en particulier entre 3 et 20 cm, en particulier entre 3 et 15 cm.
